# EUROPEAN PATENT SPECIFICATION

(11) **EP 1 482 911 B1**
(45) Date of publication and mention of the grant of the patent: **17.10.2007**
(21) Application number: 03716337.5
(22) Date of filing: 06.03.2003
(51) Int. Cl.: A61K 9/14, A23L 2/00

(54) **WATER DISPERSIBLE MIXTURE COMPRISING COMPLEX CARBOHYDRATES FROM ALOE VERA AND POLYVINYLPYRROLIDONE**
WASSERDISPERGIERBARE MISCHUNG ENTHALTEND KOMPLEXE KOHLENHYDRATE AUS ALOE VERA UND POLYVINYLPYRROLIDONE
MÉLANGE HYDRODISPERSIBLE CONTENTANT DES GLUCIDES COMPLEXES D'ALOE VERA ET POLYVINYLPYRROLIDONE

(30) Priority: 07.03.2002 US 92957
(43) Date of publication of application: 08.12.2004
(73) Proprietor: CARRINGTON LABORATORIES, INC., Irving, TX 75038-4404 (US)
(72) Inventor: YATES, Kenneth, M., Grand Prairie, TX 7050 (US); WANG, Dongmei, Irving, TX 75063 (US); LONG, John, Irving, TX 75063 (US)
(74) Representative: Fisher, Adrian John
(86) International application number: PCT/US2003/006832
(87) International publication number: WO 2003/075891

(56) References cited:
- EP-A- 0 450 141
- WO-A-95/10199
- US-A- 5 409 703
- US-A- 5 760 102

## Description

The present invention relates to a substantially dry mixture dispersible in water of which the substantially dry mixture has solid-containing complex carbohydrate from Aloe Vera and a water-soluble pharmaceutical excipient or auxiliary, selected from polyvinylpyrrolidone having a molecular weight ranging from 1000 to 30000 Daltons. and processes of preparation of the mixture.

Aloe is a tropical or subtropical plant characterized by lance-shaped leaves with jagged edges and sharp points. For centuries, this plant has been considered to have, and has been used for its, medicinal and therapeutic properties without any clear understanding or scientific analysis of the bases for these properties. Further, it is known that the biological activities associated with the fresh plant decay very rapidly once harvested and the biological effectiveness can be impacted by current methods utilized in processing.

Because of the varied activities and stability problems associated with *Aloe vera*, most methods employed for the processing of the plant result in end products that do not consistently achieve desired results. Further, Aloe leaves contain anthraquinones in its yellow sap. The anthraquinone-containing yellow sap is known to have a laxative effect with a reputation as an extremely irritating cathartic. Traditional processes for the production of various Aloe products typically involved crushing (pressure rollers), grinding (e.g., use of Thompson Aloe leaf slitter), or pressing (TCX pressure extruder) of the entire leaf of the Aloe plant to produce an Aloe vera juice, followed by various steps of filtration and stabilization of the juice. The resulting mixture is then incorporated in, or mixed with, other solutions or agents to produce the products which could be, for example, a cosmetic, a health food drink, or a topical ointment.

*Aloe vera* leaves contain a variety of chemical substances and components. Mixtures of active chemical substances of Aloe leaves have been identified, isolated and stabilized as described in U.S. Patent Nos. 4,735,935; 4,851,224; 4,917,890; 4,957,907; 4,959,214; 4,966,892; and 5,902,796, each of these is incorporated herein by reference. One group of the active chemical substances has been referred to as *Aloe vera* high molecular weight polysaccharides. Even the Aloe vera polysaccharides are made up of a mixture of polysaccharides. The term "polysaccharides" has been used loosely to include both oligomers and polymers of carbohydrates, the complex carbohydrates. A group of such complex carbohydrates has been given the name acemannan. Acemannan is an ordered linear polymer of substantially acetylated mannose monomers.

The biological, or physiological, activities of *Aloe vera* polysaccharides and their pharmaceutical applications have been the object of numerous research studies at a number of laboratories, including Carrington Laboratories. Uses of Aloe products have been described in U.S. Patent Nos. 5,106,616; 5,118,673; 5,308,838; 5,441,943; 5,443,830; 5,468,737; 5,587,364; 5,703,060; 5,760,102; 5,773,425; 5,780,453; 5,780,342; and 5,929,051, each assigned to Carrington Laboratories, Inc., the content of each of which is incorporated by reference herein. These studies have primarily focused on the activities of bioactive chemical substances of *Aloe vera* L. as antiviral agents, antitumor agents, immunostimulants, immunomodulators, vaccine adjuvants, means of reducing opportunistic infections, means of controlling inflammation, and means of stimulating wound-healing processes.

*Aloe vera* L. polysaccharides have been shown in controlled studies to increase the rate of healing in animals. These polysaccharides have also been shown to be an effective treatment for oral mucosal injuries in human clinical trials. For example, in a human clinical trial in which 20 radiotherapy/chemotherapy patients used an oral wound rinse with Aloe polysaccharides (containing acemannan) as the primary functional ingredient, the patients reported a significant decrease in their oral pain following use of the product.

However, high molecular weight carbohydrates present unique problems associated with dissolution. Due to their complex physical structure, solubility is impacted by several factors (e.g. temperature, pH, and polymer size) that affect aggregation resulting in slower time for solubilization. Therefore, most health products utilizing these materials as components exist in the soluble form as liquids, gels, creams or lotions to assure quality and consistency of the desired final product.

Improved methods for dissolution of complex carbohydrates allows for finished products to be provided as dry powders potentially improving stability of active substances. In addition, reduction in manufacturing time and improved product uniformity would result in higher quality finished products for manufacturers that potentially could impact final product cost, functionality and effectiveness.

The problems discussed above have been solved in the present invention that provides for compositions containing one or more dispersants, namely a water-soluble pharmaceutical excipient or auxiliary selected from polyvinylpyrrolidone having a molecular weight ranging from 1000 to 30000 Daltons, to disperse or dissolve the solid-containing complex carbohydrate originated, or derived, from a processed Aloe vera plant, such as *Aloe vera* leaf, *Aloe vera* gel fillet, *Aloe vera* raw gel, freeze-dried *Aloe vera* gel extract, or bulk acetylated mannan.

### SUMMARY

The present invention provides methods for dissolution of dried complex carbohydrates from Aloe Vera. This invention allows for finished products to be provided as dry powders, which potentially improves the stability of active substances. In addition, reduction in manufacturing time and improved product uniformity would result in higher quality finished products for manufacturers that potentially could impact final product cost, functionality and effectiveness.

Some of the problems associated with the dissolution of dried powders are discussed herein, and have been solved in the present invention, which provides for compositions containing one or more dispersants, namely a water-soluble pharmaceutical excipient or auxiliary selected from polyvinylpyrrolidone having a molecular weight ranging from 1000 to 30000 Daltons, to disperse or dissolve the solid-containing complex carbohydrate. The solid-containing complex carbohydrate can originate, or derive, from a processed plant, such as *Aloe vera, Aloe vera* leaf, *Aloe vera* gel fillet, *Aloe vera* raw gel, freeze-dried *Aloe vera* gel extract, or bulk acetylated mannan.

Broadly, one embodiment of the present invention pertains to a relatively dry mixture that is dispersible or soluble in water, the substantially dry mixture comprises a complex carbohydrate from Aloe Vera, and a dispersant, such as a water-soluble pharmaceutical excipient or auxiliary selected from polyvinylpyrrolidone having a molecular weight ranging from 1000 to 30000 Daltons. Other dry mixture also may include a simple sugar, a starch, or a combination thereof. The substantially dry mixture can be constituted with water, carbonated or un-carbonated, to form a final drink. The substantially dry mixture can also be in the form of a tablet or capsule.

Another object of the present invention is to provide a composition having a water-dispersible or water-soluble processed *Aloe vera* solid, which contains complex carbohydrate.

Still another object of the present invention is to provide a process to disperse or dissolve in water a processed *Aloe vera* plant.

### DETAILED DESCRIPTION

High molecular weight carbohydrates present unique problems associated with dissolution. Due to their complex physical structure, solubility is impacted by several factors such as temperature, pH, and polymer size that affect aggregation resulting in slower time for solubilization. Therefore, most health products utilizing these materials as components exist in the soluble form as liquids, gels, creams or lotions to assure quality and consistency of the desired final product.

The unique problems discussed above are inherent with complex carbohydrates, whether or not of plant origin, have been solved in the embodiments of the present invention that pertains to the production of a dry mixture comprising solid complex carbohydrate(s) from *Aloe vera*, either Aloe vera leaves or products derived or processed from *Aloe vera* leaves, and polyvinylpyrrolidone having a molecular weight ranging from 1000 to 30000 Daltons. The dry mixture can also contain a simple sugar or a starch.

*Aloe vera* gel fillet that is substantially anthraquinone-free can be produced by the following steps from a leaf of an Aloe plant:

1. Washing the Aloe leaf in a bactericidal solution to remove substantially all surface dirt and bacteria;

2. removing at least a first end portion from the washed leaf;

3. draining, preserving and collecting anthraquinone rich sap from the cut and washed leaf; and

4. removing rind from the leaf to produce a substantially anthraquinone-free gel fillet.

Aloe raw gel, "raw gel," or "Aloe juice" that is substantially anthraquinone-free having solubilized and suspended matter can be obtained by shearing and homogenizing the substantially anthraquinone-free Aloe gel fillet.

Freeze-dried *Aloe vera* gel extract containing complex carbohydrate from an Aloe leaf, such as Manapol® powder, can be produced by the following steps:

1. Obtaining Aloe raw gel, "raw gel," or "Aloe juice" having solubilized and suspended matter;

2. adding a water-soluble, lower aliphatic polar solvent, such as ethanol, to the Aloe juice to precipitate active chemical substance(s) and thereby forming a heterogeneous solution/suspension;

3. removing the water-soluble, lower aliphatic polar solvent and the solubilized matter from the heterogeneous solution to isolate the precipitated active chemical substance(s); and

4. drying, , the precipitated active chemical substance(s).

Generally, Manapol® powder, a processed *Aloe vera* solid, contains less than about 50 percent water-soluble complex carbohydrate.

Another form of freeze-dried Aloe vera gel extract containing complex carbohydrate can be produced by the following steps:

1. Obtaining Aloe raw gel, "raw gel," or "Aloe juice" having solubilized and suspended matter;

2. adjusting the pH of the Aloe juice;

3. adding a water-soluble, lower aliphatic polar solvent, such as ethanol, to the Aloe juice to precipitate active chemical substance(s) and thereby forming a heterogeneous solution/suspension;

4. removing the water-soluble, lower aliphatic polar solvent and the solubilized matter from the heterogeneous solution to isolate the precipitated active chemical substance(s); and

5. drying, , the precipitated active chemical substance(s).

Generally, "bulk acetylated mannan" ("BAM") may be prepared from Aloe leaves as follows:

1. Aloe leaves are washed, sliced open and filleted to remove the leaf rind. The clean (substantially anthraquinones free) inner gel is retained while the green rind is discarded.

2. The filleted material is homogenized and extensively filtered to remove most of the pulp.

3. The clear viscous gel is acidified.

4. The acidified gel is then extracted with 95 percent ethanol at controlled temperature. The solid precipitate is collected. The alcohol extraction process eliminates most alcohol/water-soluble substances such as organic acids, oligosaccharides, monosaccharides, anthraquinones and inorganic salts.

5. The solid *Aloe vera* extract is then dried, and milled to a white powder. The product at this stage still contains some moisture, monosaccharides, oligosaccharides, protein, organic/inorganic salts and other substances. The product can be stored as a source of BAM. The product is stable at room temperature in the dried form for several years if protected from additional moisture. Generally, processed *Aloe vera* solid of BAM grade contains more than about 50 percent water-soluble complex carbohydrate.

The detailed procedures for producing substantially anthraquinone-free Aloe gel, for producing substantially anthraquinone-free Aloe juice, for extracting active chemical substance(s) from an Aloe leave, for preparing BAM and for extracting from an Aloe leave substantially nondegradable lyophilized ordered linear polymer of mannoses have been described in Carrington's U.S. Patent Nos. 4,735,935; 4,851,224; 4,917,890; 4,957,907; 4,959,214; 4,966,892; and 5,902,796, the entire content of each of which is incorporated by reference. The uses of Aloe products have been described in Carrington's various U.S. patents mentioned above.

The dispersant of water-soluble pharmaceutical excipient or auxiliary for this invention includes polyvinylpyrrolidone ("PVP" or povidone). The weight average molecular weight ("MW") of PVP can be from 1,500 to 30,000; the preferred MW range is from 2,000 to 20,000; and the more preferred MW range is from 2,000 to 15,000. Based on weight, the w/w% ratio of the water-soluble pharmaceutical excipient or auxiliary to the solid-containing complex carbohydrate varies from 1:2 to about 1:100. Preferably, the weight ratio of the solid-containing complex carbohydrate to the water-soluble pharmaceutical auxiliary ranges from about 1:10 to about 1:40; and more preferably, the weight ratio of the solid-containing complex carbohydrate to the water-soluble pharmaceutical auxiliary range from about 1:2 to about 1:10.

A additional dispersant to the dry mixture of this invention includes a simple sugar. Examples of simple sugar include fructose, sucrose, lactose, glucose, maltose, dextrose, and others. The preferred simple sugar is fructose. Based on weight, the w/w% ratio of the simple sugar to the solid-containing carbohydrate can vary from about 40:1 to about 1:1; preferably from about 3:1 to about 20:1; and more preferably from about 10:1 to about 5:1.

Another additional dispersant includes starck. The starch includes maltodextran, cornstarch, rice flour, amylose, and others. The preferred starch is maltodextran. Based on weight, the w/w% ratio of the starch to the solid-containing complex carbohydrate can vary from about 40:1 to about 5:1. Preferably, the weight ratio of the starch to the solid-containing complex carbohydrate varies from about 20:1 to about 10:1; and more preferably, the weight ratio of the water-soluble pharmaceutical auxiliary to the solid-containing complex carbohydrate ranges from about 10:1 to about 2:1.

Another optional ingredient usable in this invention is a preservative. Examples of the preservative include benzalkonium chloride, methylparaben, potassium sorbate, sodium benzoate, imidazolidinyl urea, and others. The amount of the preservative used can range from about 0.01 weight percent to about 2 weight percent, based on the total weight of the final product.

Still another optional ingredient that may be used for the present invention is a flavoring additive. Such flavoring additives include spicy flavors, such as cinnamon or anis; fruity flavors, such as citrus fruits or extracts; botanical flavors, such as rose hip or vanilla; and synthetic flavorants. Flavorants may be derived from the natural edible fruits, spices and plants or from synthetically prepared flavors made to simulate natural flavorants. The amount of the flavorant used depends upon the flavor or flavors selected, the flavor impression desired, and the form of the flavor additive used. Commonly when a concentrated flavorant is used, the amount of flavorant added may vary from about 0.001 to about 10 percent by weight, based on the total weight of the product. Alternatively, a fruit-flavored drink, a health drink, a sport drink and/or a natural vegetable or fruit juice, either dilute or concentrated, can be added to the product of the present invention. The amount will depend on the desired flavor and taste.

Non-caloric or low-caloric sweeteners can also be used. The low-caloric sweeteners can be derived either from natural origins or from synthetic sources. Examples of such non-caloric sweeteners can be derived either from natural origins or from synthetic sources. Examples of such non-caloric or low-caloric sweeteners include, but are not limited to, saccharin, cyclamates, acetosulfam, sorbitol, xylitol, L-aspartyl-L-phenyl-alanine ester (e.g. aspartame), and others. The amount of the non-caloric sweetener used depends on the particular sweetener, or mixture of sweeteners, and the sweetness intensity desired. Generally, the non-caloric or low-caloric sweetener ranges from about 0.0001 to about 1 weight percent, based on the total weight of the product.

In preparing the substantially dry mixture dispersible, or soluble, in water of the present invention, a predetermined amount of the processed Aloe vera plant solid, which contains complex carbohydrate, is mixed with a predetermined amount of a dispersant. Other dispersant, or optional ingredient, or both, can also be added to the mixture. The resultant product, the substantially dry mixture can be constituted with water, carbonated or uncarbonated to form a drink. The substantially dry mixture can be in the form of a powder, tablet or capsules.

### EXAMPLE 1

Time required for the dissolution in water of a mixture of 10 mg of micronized BAM powder and different types of PVP.

| Type of PVP | Wt. Ratio of BAM: PVP | Time of Dissolution |
|---|---|---|
| C 15 | 1:5 | 45 sec |
| | 1:10 | 35 sec |
| | 1:20 | 29 sec |
| | 1:40 | 25 sec |
| | | |
| K 29/32 | 1:5 | 48 sec |
| (Comparative example - not part of invention) | 1:10 | 43 sec |
| | 1:20 | 1 min |
| | 1:40 | 1 min 5 sec |

To each mixture in a beaker was added 2 ml of water and stirred with a stirring rod until the powder had dissolved.

### EXAMPLE 2

Time required for the dissolution in water of a mixture of 10 mg of micronized BAM powder, C15 PVP, and maltodextran.

| Wt. Ratio of BAM: PVP: Maltodextran | Time of Dissolution |
|---|---|
| 1:20:5 | 19 sec |
| 1:20:10 | 25 sec |
| 1:20:20 | 20 sec |
| | |
| 1:40:5 | 22 sec |
| 1:40:10 | 17 sec |
| 1:40:20 | 21 sec |

To each mixture in a beaker was added 2 ml of water and stirred with a stirring rod until the mixture had dissolved.

### EXAMPLE 3

Time required for the dissolution of 10 mg of micronized BAM powder, C15 PVP, maltodextran, and fructose.

| Wt. Ratio of BAM: PVP: Maltodextran: Fructose | Time of Dissolution |
|---|---|
| 1:20:5:2 | 31 sec |
| 1:20:5:5 | 35 sec |
| 1:20:5:10 | 25 sec |
| | |
| 1:40:10:2 | 33 sec |
| 1:40:10:5 | 25 sec |
| 1:40:10:10 | 27 sec |

To each mixture in the beaker was added 2 ml of water and stirred with a stirring rod until the mixture had dissolved.

### EXAMPLE 4

Time required for the dissolution of mixtures of 1.05 grams of BAM, C15 PVP, maltodextran, and fructose.

| Wt. Ratio of BAM: C 15 PVP: Maltodextran; Fructose | Result |
|---|---|
| 1:20:5:10 | immediate dispersion - 30 sec. of shaking |
| 1:20:5:0 | residue on the bottom after 2 mins. of shaking |
| 1:40:10:10 | residue left - unacceptable |
| | |
| 1:10:5:10 | immediate dispersion when shaken but not stirred |

It is clear that the present invention is well adapted to carry out the objects and advantages mentioned herein as well as those inherent in the invention.

It should be appreciated by those of ordinary skill in the art that other embodiments may incorporate the concepts, methods, precursors, and compositions of the above description and examples. The description and examples contained herein are not intended to limit the scope of the invention, but are included for illustration purposes only. It is to be understood that other embodiments of the invention can be developed and fall within the scope of the invention and claims.

## Claims

1. A substantially dry mixture dispersible in water comprising: a weight/weight% ratio of a Aloe vera solid having complex carbohydrate to a polyvinylpyrrolidone, wherein the weight/weight% ratio ranges from 1:2 to 1:100, and wherein the polyvinylpyrrolidone has a molecular weight ranging from 1,000 to 30,000 Dalton.

2. The substantially dry mixture of claim 1, wherein the weight/weight% ratio of the Aloe vera solid having complex carbohydrate to the polyvinylpyrrolidone ranges from 1:5 to 1:40.

3. The substantially dry mixture of claim 1 additionally comprising maltodextran comprising: a weight/weight% ratio of a Aloe vera solid having complex carbohydrate to a polyvinylpyrrolidone to maltodextran, wherein the weight/weight% ratio ranges from 1:10:5 to 1:40:20.

4. The substantially dry mixture of claim 3 additionally comprising a fructose comprising: a weight/weight ratio of a Aloe vera solid having complex carbohydrate to a polyvinylpyrrolidone to a maltodextran to a fructose, wherein the weight/weight% ratio ranges from 1:10:5:2 to 1:40:20:10.

5. The substantially dry mixture of claim 4, wherein the weight/weight% ratio of the Aloe vera solid having complex carbohydrate to the polyvinylpyrrolidone to the maltodextran to the fructose is ranges from 1:10:5:10.

6. A method for dispersing in water a complex carbohydrate derived from a processed Aloe vera plant, the method comprising:
(a) mixing the complex carbohydrate derived from the processed plant with polyvinylpyrrolidone to give a mixture, and wherein the polyvinylpyrrolidone has a weighted average molecular weight ranging from 1,000 to 30,000 Dalton and wherein the weight/weight % ratio of an Aloe vera solid having complex carbohydrate to a polyvinylpyrrolidone ranges from 1:2 to 1:100; and
(b) adding water to the mixture.

7. The method of claim 6, wherein the polyvinylpyrrolidone has a weighted average molecular weight ranging from 1,000 to 20,000 Dalton.

8. The method of claim 6 further comprising adding to the mixture a simple sugar, a starch, or a mixture thereof.

9. The method of claim 8, wherein the simple sugar comprises fructose, sucrose, lactose, glucose, or a mixture thereof.

10. The method of claim 8, wherein the starch comprises maltodextran, corn starch, rice flour, amylose, or a mixture thereof.

11. The substantially dry mixture of claim 1, further comprising a simple sugar, a starch, or a mixture thereof.

12. The substantially dry mixture of claim 11, wherein the weight/weight% ratio of the processed Aloe vera solid to the polyvinylpyrrolidone ranges from 1:10 to 1:40.

13. The substantially dry mixture of any of claims 1, 3, 4 and 11, wherein the processed Aloe vera solid comprises more than 50 percent of water-soluble complex carbohydrate.

14. The substantially dry mixture of any of claims 1, 3, 4 and 11, wherein the processed Aloe vera solid comprises less than 50 percent of water-soluble complex carbohydrate.

15. The substantially dry mixture of claim 11, wherein the simple sugar comprises fructose, sucrose, lactose, glucose, or a mixture thereof.

16. The substantially dry mixture of claim 11 wherein the starch comprises maltodextran, corn starch, rice flour, amylose, or a mixture thereof.

17. The substantially dry mixture of any of claims 1, 3, 4 and 11, wherein the substantially dry mixture is in the form of a powder suitable for constituting with water, carbonated or uncarbonated, to form a final drink.

18. The substantially dry mixture of any of claims 1, 3, 4 and 11, wherein the substantially dry mixture is in the form of a powder suitable for constituting with water, carbonated or uncarbonated, to form a final mixture.

19. The substantially dry mixture of any of claims 1, 3, 4 and 11, wherein the substantially dry mixture is in the form of a tablet or capsule.

20. The tablet or capsule of claim 19 wherein the tablet or capsule is capable of rapidly dissolving in water.

## Patentansprüche

1. Im wesentlichen trockenes Gemisch, dispergierbar in Wasser, umfassend: ein Gew./Gew.-%-Verhältnis an Aloe-Vera-Feststoff mit komplexem Kohlenhydrat zu einem Polyvinylpyrrolidon, wobei das Gew./Gew.-%-Verhältnis von 1:2 bis 1:100 reicht, und wobei das Polyvinylpyrrolidon ein Molekulargewicht im Bereich von 1.000 bis 30.000 Dalton aufweist.

2. Im wesentlichen trockenes Gemisch gemäß Anspruch 1, wobei das Gew./Gew.-%-Verhältnis des Aloe-Vera-Feststoffs mit komplexem Kohlenhydrat zu dem Polyvinylpyrrolidon von 1:5 bis 1:40 reicht.

3. Im wesentlichen trockenes Gemisch gemäß Anspruch 1, zusätzlich umfassend Maltodextran, umfassend ein Gew./Gew.-%-Verhältnis von einem Aloe-Vera-Feststoff mit komplexem Kohlenhydrat zu einem Polyvinylpyrrolidon zu Maltodextran, wobei das Gew./Gew.-%-Verhältnis von 1:10:5 bis 1:40:20 reicht.

4. Im wesentlichen trockenes Gemisch gemäß Anspruch 3, zusätzlich umfassend eine Fructose, umfassend: ein Gew./Gew.-%-Verhältnis von einem Aloe-Vera-Feststoff mit komplexem Kohlenhydrat zu einem Polyvinylpyrrolidon zu einem Maltodextran zu einer Fructose, wobei das Gew./Gew.-%-Verhältnis von 1:10:5:2 bis 1:40:20:10 reicht.

5. Im wesentlichen trockenes Gemisch gemäß Anspruch 4, wobei das Gew./Gew.-%-Verhältnis von dem Aloe-Vera-Feststoff mit komplexem Kohlenhydrat zu dem Polyvinylpyrrolidon zu dem Maltodextran zu der Fructose

6. Verfahren zum Dispergieren eines komplexen Kohlenhydrats, abgeleitet von einer verarbeiteten Aloe-Vera-Pflanze, in Wasser, wobei das Verfahren umfaßt:
(a) das Mischen des komplexen Kohlenhydrats, abgeleitet von der verarbeiteten Pflanze, mit Polyvinylpyrrolidon unter Erhalten eines Gemisches, und wobei das Polyvinylpyrrolidon ein gewichtsmittleres Molekulargewicht im Bereich von 1.000 bis 30.000 Dalton aufweist, und wobei das Gew./Gew.-%-Verhältnis von einem Aloe-Vera-Feststoff mit komplexem Kohlenhydrat zu einem Polyvinylpyrrolidon von 1:2 bis 1:100 reicht, und
(b) das Zugeben von Wasser zu dem Gemisch.

7. Verfahren gemäß Anspruch 6, wobei das Polyvinylpyrrolidon ein gewichtsmittleres Molekulargewicht im Bereich von 1.000 bis 20.000 Dalton aufweist.

8. Verfahren gemäß Anspruch 6, weiter umfassend das Zugeben eines einfachen Zuckers, einer Stärke oder eines Gemisches davon zu dem Gemisch.

9. Verfahren gemäß Anspruch 8, wobei der einfache Zucker Fructose, Sucrose, Lactose, Glucose oder ein Gemisch davon umfaßt.

10. Verfahren gemäß Anspruch 8, wobei die Stärke Maltodextran, Maisstärke, Reismehl, Amylose oder ein Gemisch davon umfaßt.

11. Im wesentlichen trockenes Gemisch gemäß Anspruch 1, weiter umfassend einen einfachen Zucker, eine Stärke oder ein Gemisch davon.

12. Im wesentlichen trockenes Gemisch gemäß Anspruch 11, wobei das Gew./Gew.-%-Verhältnis des verarbeiteten Aloe-Vera-Feststoffs zu dem Polyvinylpyrrolidon von 1:10 bis 1:40 reicht.

13. Im wesentlichen trockenes Gemisch gemäß einem der Ansprüche 1, 3, 4 und 11, wobei der verarbeitete Aloe-Vera-Feststoff mehr als 50% wasserlösliches komplexes Kohlenhydrat umfaßt.

14. Im wesentlichen trockenes Gemisch gemäß einem der Ansprüche 1, 3, 4 und 11, wobei der verarbeitete Aloe-Vera-Feststoff weniger als 50% wasserlösliches komplexes Kohlenhydrat umfaßt.

15. Im wesentlichen trockenes Gemisch gemäß Anspruch 11, wobei der einfache Zucker Fructose, Sucrose, Lactose, Glucose oder ein Gemisch davon umfaßt.

16. Im wesentlichen trockenes Gemisch gemäß Anspruch 11, wobei die Stärke Maltodextran, Maisstärke, Reismehl, Amylose oder ein Gemisch davon umfaßt.

17. Im wesentlichen trockenes Gemisch gemäß einem der Ansprüche 1, 3, 4 und 11, wobei das im wesentlichen trockene Gemisch in der Form eines Pulvers, geeignet zum Konstituieren mit Wasser, carbonisiert oder uncarbonisiert, zur Bildung eines Fertiggetränks ist.

18. Im wesentlichen trockenes Gemisch gemäß einem der Ansprüche 1, 3, 4 und 11, wobei das im wesentlichen trockene Gemisch in der Form eines Pulvers, geeignet zum Konstituieren mit Wasser, carbonisiert oder uncarbonisiert, zur Bildung eines Fertiggemisches ist.

19. Im wesentlichen trockenes Gemisch gemäß einem der Ansprüche 1, 3, 4 und 11, wobei das im wesentlichen trockene Gemisch in der Form einer Tablette oder Kapsel ist.

20. Tablette oder Kapsel gemäß Anspruch 19, wobei die Tablette oder Kapsel befähigt ist, rasch in Wasser gelöst zu werden.

## Revendications

1. Mélange substantiellement sec dispersable dans l'eau, comprenant: un rapport de % poids/poids d'un solide d'Aloe vera comportant un glucide complexe à une polyvinylpyrrolidone, où le rapport de % poids/poids va de 1:2 à 1:100, et où la polyvinylpyrrolidone a une masse moléculaire allant de 1000 à 30 000 Dalton.

2. Mélange substantiellement sec selon la revendication 1, dans lequel le rapport de % poids/poids du solide d'Aloe vera comportant un glucide complexe à la polyvinylpyrrolidone va de 1:5 à 1:40.

3. Mélange substantiellement sec selon la revendication 1, comprenant en outre du maltodextrane et comprenant: un rapport de % poids/poids d'un solide d'Aloe vera comportant un glucide complexe à une polyvinylpyrrolidone à un maltodextrane, où le rapport de % poids/poids va de 1:10:5 à 1:40:20.

4. Mélange substantiellement sec selon la revendication 3, comprenant en outre un fructose et comprenant: un rapport de % poids/poids d'un solide d'Aloe vera comportant un glucide complexe à une polyvinylpyrrolidone à un maltodextrane à un fructose, où le rapport de % poids/poids va de 1:10:5:2 à 1:40:20:10.

5. Mélange substantiellement sec selon la revendication 4, dans lequel le rapport de % poids/poids du solide d'Aloe vera comportant un glucide complexe à la polyvinylpyrrolidone au maltodextrane au fructose est de 1:10:5:10.

6. Procédé pour disperser dans l'eau un glucide complexe dérivé d'une plante Aloe vera transformée, le procédé comprenant:
(a) le mélange du glucide complexe dérivé de la plante transformée avec de la polyvinylpyrrolidone pour donner un mélange, et dans lequel la polyvinylpyrrolidone a une masse moléculaire moyenne pondérée allant de 1000 à 30 000 Dalton et dans lequel le rapport de % poids/poids d'un solide d'Aloe vera comportant un glucide complexe à une polyvinylpyrrolidone va de 1:2 à 1:100; et
(b) l'addition d'eau au mélange.

7. Procédé selon la revendication 6, dans lequel la polyvinylpyrrolidone a une masse moléculaire moyenne pondérée allant de 1000 à 20 000 Dalton.

8. Procédé selon la revendication 6, comprenant en outre l'addition au mélange d'un sucre simple, d'un amidon, ou d'un mélange de ceux-ci.

9. Procédé selon la revendication 8, dans lequel le sucre simple comprend du fructose, du saccharose, du lactose, du glucose, ou un de leurs mélanges.

10. Procédé selon la revendication 8, dans lequel l'amidon comprend du maltodextrane, de l'amidon de maïs, de la farine de riz, de l'amylose ou un de leurs mélanges.

11. Mélange substantiellement sec selon la revendication 1, comprenant en outre un sucre simple, un amidon ou un mélange de ceux-ci.

12. Mélange substantiellement sec selon la revendication 11, dans lequel le rapport de % poids/poids du solide d'Aloe vera transformé à la polyvinylpyrrolidone va de 1:10 à 1:40.

13. Mélange substantiellement sec selon l'une quelconque des revendications 1, 3, 4 et 11, dans lequel le solide d'Aloe vera transformé comprend plus de 50 pour cent d'un glucide complexe hydrosoluble.

14. Mélange substantiellement sec selon l'une quelconque des revendications 1, 3, 4 et 11, dans lequel le solide d'Aloe vera transformé comprend moins de 50 pour cent de glucide complexe hydrosoluble.

15. Mélange substantiellement sec selon la revendication 11, dans lequel le sucre simple comprend du fructose, du saccharose, du lactose, du glucose ou un de leurs mélanges.

16. Mélange substantiellement sec selon la revendication 11, dans lequel l'amidon comprend du maltodextrane, de l'amidon de maïs, de la farine de riz, de l'amylose ou un de leurs mélanges.

17. Mélange substantiellement sec selon l'une quelconque des revendications 1, 3, 4 et 11, dans lequel le mélange substantiellement sec se présente sous forme d'une poudre reconstituable avec de l'eau, carbonatée ou non carbonatée, pour former une boisson finale.

18. Mélange substantiellement sec selon l'une quelconque des revendications 1, 3, 4 et 11, dans lequel le mélange substantiellement sec se présente sous forme d'une poudre reconstituable avec de l'eau, carbonatée ou non carbonatée, pour former un mélange final.

19. Mélange substantiellement sec selon l'une quelconque des revendications 1, 3, 4 et 11, dans lequel le mélange substantiellement sec se présente sous la forme d'un comprimé ou d'une capsule.

20. Comprimé ou capsule selon la revendication 19, où le comprimé ou la capsule est susceptible de se dissoudre rapidement dans l'eau.
